Europäisches Patentamt

European Patent Office

Office européen des brevets

(19)

(11) Veröffentlichungsnummer: **0 195 334**
**B1**

(12)

# EUROPÄISCHE PATENTSCHRIFT

(45) Veröffentlichungstag der Patentschrift:
24.10.90

(51) Int. Cl.⁵: **A61K 35/78, A23L 1/076**

(21) Anmeldenummer: **86103117.7**

(22) Anmeldetag: **08.03.86**

(54) Verfahren zum Aufschliessen von Blütenpollen.

(30) Priorität: **19.03.85 DE 3509759**

(43) Veröffentlichungstag der Anmeldung:
**24.09.86 Patentblatt 86/39**

(45) Bekanntmachung des Hinweises auf die Patenterteilung:
**24.10.90 Patentblatt 90/43**

(84) Benannte Vertragsstaaten:
**AT BE CH DE FR GB IT LI LU NL SE**

(56) Entgegenhaltungen:
**DE-A- 2 408 996**
**DE-A- 2 632 045**
**JP-A-60 024 169**
**US-A- 4 588 134**

(73) Patentinhaber: **KOHLENSÄUREWERK DEUTSCHLAND GMBH, Melkenweg 1, D-5462 Bad Hönningen(DE)**

(72) Erfinder: **Grünhoff, Ulrich, Dipl.-Ing., Marktstrasse 12, D-5462 Bad Hönningen(DE)**
Erfinder: **Urbat, Lutz, Dipl.-Ing., Friedlandstrasse 23, D-5462 Bad Hönningen(DE)**

(74) Vertreter: **Lauer, Dieter, Dr., c/o Kali-Chemie Aktiengesellschaft Postfach 220, D-3000 Hannover 1(DE)**

## Beschreibung

Die Erfindung betrifft ein Verfahren zum Aufschließen von Blütenpollen sowie eine Vorrichtung zur Durchführung dieses Verfahrens.

Blütenpollenzubereitungen finden auf Grund ihres Gehalts an wertvollen Inhaltsstoffen vielfältige Anwendung als Revitalisierungs-, Stärkungs- und Heilmittel. Bei direkter Verabreichung von Pollen wird aber nur eine sehr geringe Bioverfügbarkeit erreicht, da das einzelne Pollenkorn von einer widerstandsfähigen Außenhaut (Speroderm) umgeben ist, die vom menschlichen Verdauungssystem kaum angegriffen wird. Es ist zwar möglich, die Außenhaut durch chemische Reaktionen abzubauen und so die Inhaltsstoffe zu gewinnen. Dieser Weg ist aber unbefriedigend, da z. B. Rückstände der Chemikalien im Endprodukt verbleiben, eine Wertminderung durch Verlust wertvoller Inhaltsstoffe eintritt und auch Geschmacksveränderungen auftreten.

Ein anderer Versuch, die Pollenhaut zu zerstören ist, die Pollen mit einem Kaltmahlverfahren aufzuschließen (DE-PS 29 22 485). Auch dieses Verfahren weist Nachteile auf. Zum einen ist der erreichte Aufschlußgrad noch nicht befriedigend, zum anderen wird ein Pulver erhalten, das nach kurzer Lagerung zu harten Massen erstarrt, die erneut gebrochen oder gemahlen werden müssen.

Aufgabe der Erfindung ist es, ein Verfahren zum Aufschließen von Blütenpollen zur Verfügung zu stellen, das die Nachteile des Standes der Technik überwindet.

Eine weitere Aufgabe besteht darin, eine geeignete Vorrichtung zur Durchführung des Verfahrens zur Verfügung zu stellen.

Die Aufgaben werden gelöst, durch die in den Ansprüchen angegebenen Verfahren und die Vorrichtung.

Die Erfindung umfaßt also ein Verfahren zum Aufschließen von Blütenpollen, dadurch gekennzeichnet, daß man in einer ersten Stufe Blütenpollen einer Trocknung unterwirft und anschließend die getrockneten Blütenpollen einer an sich bekannten Druckzerkleinerungsbehandlung unterwirft.

Die Trocknung in der ersten Stufe soll schonend erfolgen. Durch die Trocknung wird der Feuchtigkeitsgehalt der Pollenkörner auf höchstens 8 Gew.-%, vorzugsweise auf 1 - 5 Gew.-% eingestellt. Die Trocknungsenergie ist dabei derart abzustimmen, daß die Temperatur des zu trocknenden Gutes 50 °C nicht überschreitet.

Geeignete schonende Trocknungsverfahren sind an sich bekannt. Insbesondere geeignet sind die Vakuumtrocknung, Lyophilisierung oder Mikrowellentrocknung. Besonders geeignet für die erfindungsgemäße Kombination ist die Mikrowellentrocknung. Bei diesem Trockenverfahren sind Trocknungszeiten von bis zu 5 Minuten ausreichend, um den gewünschten Feuchtigkeitsgehalt einzustellen.

Die Pollen können nach der Trocknung noch gekühlt werden. Diese Maßnahme ist besonders zu empfehlen, wenn bis an die angegebene Temperaturobergrenze erhitzt wurde und/oder die nachfolgende Druckzerkleinerungsbehandlung nicht mit Inertgasen vorgenommen wird.

Die als Stufe b) vorzunehmende Druckzerkleinerungsbehandlung (cell-cracking Verfahren) ist an sich bekannt und wird beispielsweise beschrieben in FR-A 2 030 019, DE-OS 26 32 045 oder in DE-OS 33 47 152.

Die Druckzerkleinerungsbehandlung besteht im wesentlichen daraus, das zu zerkleinernde Gut in einer Druckkammer mit einem Druckgas zu beaufschlagen und über eine Entspannungsvorrichtung, z. B. einem Entspannungsventil, in eine Entspannungskammer schlagartig zu entleeren. In der Entspannungskammer können Einbauten wie Leitbleche oder Prallkopf etc. in an sich bekannter Weise angeordnet sein.

Als Druckgas können technisch übliche Gase verwendet werden. Bevorzugt wird die Verwendung inerter Gase, also solcher Gase, die mit dem Pollenmaterial nicht in unerwünschter Weise reagieren. Solche Gase sind beispielsweise Kohlendioxid, Stickstoff, Edelgase, Schwefelhexafluorid, bei Raumtemperatur gasförmige Fluor-Chlor-Kohlenwasserstoffe, Methan, Äthan, Propan etc. Besonders bevorzugt ist die Verwendung von Kohlendioxid als Druckgas.

Die bei der Druckbehandlung aufzuwendenden Drucke betragen mindestens 30 bar, vorzugsweise mindestens 40 bar.

Im Anschluß an die Druckzerkleinerung können weitere, an sich bekannte Verfahrensschritte wie Trennoperationen (z. B. Siebung, Windsichten etc.) angeschlossen sein.

Es ist auch möglich, den in der einstufigen Behandlung erreichten Zerkleinerungsgrad zu verbessern, sei es durch eine mehrstufige Behandlung, sei es durch Rückführung grober Anteile.

Überraschenderweise hat sich herausgestellt, daß die angegebene Kombination von Verfahrensschritten gute Ergebnisse liefert. Vorversuche, Blütenpollen allein durch Druckzerkleinerung aufzuschließen brachten hinsichtlich des Aufschlusses zwar oft gute Resultate, allerdings wurde auch hier ein Verbacken der aufgeschlossenen Pollen beobachtet.

Nach dem erfindungsgemäßen Verfahren resultiert ein gut aufgeschlossenes sensorisch unverändertes, lagerstabiles und gut rieselfähiges Pollenprodukt, das bei Verwendung von Inertgasen, insbesondere von $CO_2$ als Druckgas, auch gleich unter Schutzgas verpackt werden kann. Bei Verwendung von $CO_2$ kann außerdem durch Variation der Dichte eine die Inhaltsstoffe besonders schonende Druckzerkleinerung durchgeführt werden bzw. ein besonders gutes Aufschlußergebnis erzielt werden.

Die Erfindung betrifft auch eine Vorrichtung zur Durchführung des erfindungsgemäßen Verfahrens.

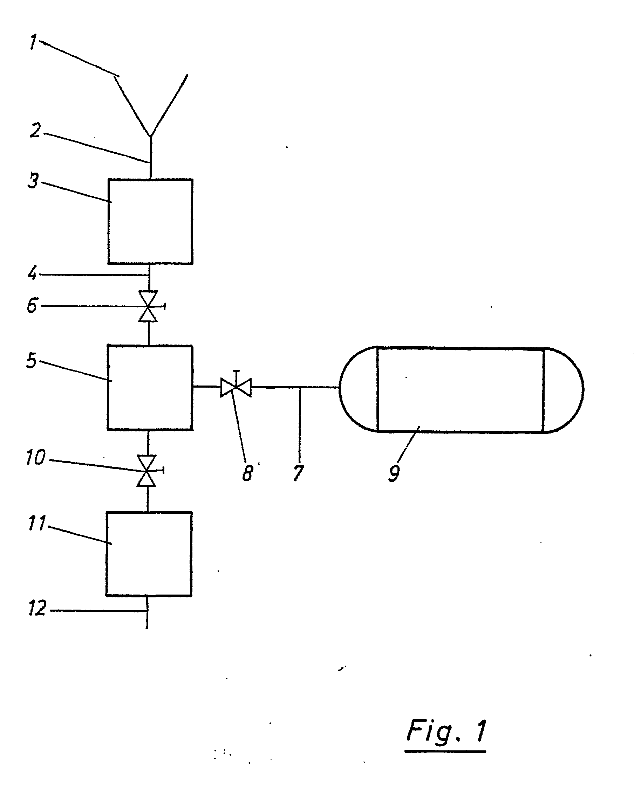

Diese ist in Figur 1 schematisch dargestellt.

Über eine Dosiervorrichtung (1) und Leitung (2) wird Blütenpollen der Trockenstufe (3) zugeführt. In der bevorzugten Variante ist dieses eine Mikrowellen-Trockenstation. Aus der Trockenstufe (3) wird der getrocknete Blütenpollen über Leitung (4) der Druckkammer (5) zugeführt. Danach wird das Absperrventil (6) geschlossen und Druckgas über Leitung (7) und Ventil (8) aus dem Reservoir (9) aufgedrückt. Nach Verschließen des Ventils (8) wird das Druckentspannungsventil (10) geöffnet und der Inhalt der Druckkammer (5) in die Entspannungskammer (11) entleert. Das aufgeschlossene Produkt kann über Leitung (12) entnommen werden.

In weiteren Ausgestaltungen der erfindungsgemäßen Vorrichtung können vorgesehen sein

- eine zweite Druckkammer oder weitere Druckkammern (5a, ...); die Druckkammern (5a ...) sind entsprechend der Druckkammer (5) mit Zuleitungen (4a, ...), (7a, ...), Absperrorganen (8a, ...) und Druckentlastungsventilen (10a) ...) versehen und sind zwischen Druckkammer (5) und Entspannungskammer (11) angeordnet. Jede nachfolgende Druckkammer dient dabei gleichzeitig als Entspannungskammer für die vorangehende Druckkammer; (10a) ...) versehen und sind zwischen Druckkammer (5) und Entspannungskammer (11) angeordnet, Jede nachfolgende Druckkammer dient dabei gleichzeitig als Entspannungskammer für die vorangehende Druckkammer;
- Rückführungsleitung zur Druckkammer für Grobanteile
- Einbauten wie Leitbleche, Prallkopf etc. in die Entspannungskammer (11).

Das folgende Beispiel soll die Erfindung näher charakterisieren, ohne ihren Schutzumfang zu begrenzen.

Beispiel

In einer Vorrichtung entsprechend Figur 1 wurden Blütenpollen aufgeschlossen. Der Feuchtigkeitsgehalt des Einsatzmaterials betrug 12 bis 15 Gew.-%. Nach Trocknung durch Mikrowellenbehandlung (Guttemperatur ca. 50 °C, Verweilzeit ca. 2 Minuten) betrug der Feuchtigkeitsgehalt 5 Gew.-%.

Im Anschluß an die Trocknung wurde eine 2-stufige Druckzerkleinerung ($CO_2$, 50 bar) durchgeführt. Nach der ersten Stufe wurde ein Feinkornanteil (< 150 µ) von 80 Gew.-%, nach der zweiten Stufe von 91 Gew.-% erhalten. In einem Vergleichsversuch, in dem in ansonsten gleicher Weise ohne Trocknung gearbeitet wurde, wurden 35 Gew.-% bzw. 48 Gew.-% Feinanteile erzielt.

Das erfindungsgemäße Produkt blieb nach der Verpackung rieselfähig, während das Produkt des Vergleichsversuches bereits nach ca. 1 Stunde kompakt wurde.

**Patentansprüche**

1. Verfahren zum Aufschließen von Blütenpollen, dadurch gekennzeichnet, daß man
   a) in einer ersten Stufe Blütenpollen einer Trocknung unterwirft und anschließend
   b) die getrockneten Blütenpollen einer an sich bekannten Druckzerkleinerungsbehandlung unterwirft.

2. Verfahren nach Anspruch 1, dadurch gekennzeichnet, daß man in Stufe a) den Feuchtigkeitsgehalt der Pollen auf höchstens 8 % einstellt.

3. Verfahren nach einem der Ansprüche 1 oder 2, dadurch gekennzeichnet, daß die Temperatur des zu trocknenden Gutes 50 °C nicht überschreitet.

4. Verfahren nach einem der vorhergehenden Ansprüche, dadurch gekennzeichnet, daß man als Trocknungsmethode eine an sich bekannte Vakuumtrocknung, Lyophilisierung oder Mikrowellentrocknung vorsieht.

5. Verfahren nach Anspruch 4, dadurch gekennzeichnet, daß man eine Mikrowellentrocknung vorsieht.

6. Verfahren nach Anspruch 5, dadurch gekennzeichnet, daß man die Trocknung bis zu 5 Minuten lang durchführt.

7. Verfahren nach einem der vorhergehenden Ansprüche, dadurch gekennzeichnet, daß man die Druckzerkleinerung gemäß Stufe b) mittels inerter Gase durchführt.

8. Verfahren nach Anspruch 7, dadurch gekennzeichnet, daß man als inertes Gas Kohlendioxid verwendet.

9. Verfahren nach einem der vorhergehenden Ansprüche, dadurch gekennzeichnet, daß man die Druckzerkleinerung unter einem Druck von mindestens 30 bar, vorzugsweise mindestens 40 bar durchführt.

10. Verfahren nach einem der vorhergehenden Ansprüche, dadurch gekennzeichnet, daß man das zu zerkleinernde Gut in einer Druckkammer mit dem Druckgas beaufschlagt und über ein Entspannungsventil gegen einen, in einer Entspannungskammer angeordneten Prallkopf entleert.

11. Verfahren nach einem der vorhergehenden Ansprüche, dadurch gekennzeichnet, daß man die Druckzerkleinerung mehrstufig durchführt.

12. Vorrichtung zur Durchführung von Verfahren gemäß einem der vorhergehenden Ansprüche, gekennzeichnet durch mindestens eine Druckkammer (5), Zuleitungen für Zerkleinerungsgut (4) und Druckgas (7), Absperrorgane (6, 8) Entspannungskammer (11) und Druckentlastungsventil (10) sowie zusätzlich einer Trockenstation (3), vorzugsweise Mikrowellen-Trockenstation, die der Druckkammer (5) vorgeschaltet ist.

13. Vorrichtung nach Anspruch 12, dadurch gekennzeichnet, daß in der Entspannungskammer (11) ein Prallkopf angeordnet ist.

**Claims**

1. A method of breaking up pollen, characterised in that
   a) in a first stage pollen is subjected to drying and then
   b) the dried pollen is subjected to a pressure comminution treatment known per se.

2. A method according to Claim 1, characterised in that in stage a) the moisture content of the pollen is set to a maximum of 8%.

3. A method according to one of Claims 1 or 2, characterised in that the temperature of the material which is to be dried does not exceed 50°C.

4. A method according to one of the preceding Claims, characterised in that provision is made for vacuum drying, lyophilisation or microwave drying which is known per se as the drying method.

5. A method according to Claim 4, characterised in that provision is made for microwave drying.

6. A method according to Claim 5, characterised in that the drying is carried out for up to 5 minutes.

7. A method according to one of the preceding Claims, characterised in that the pressure comminution treatment of stage b) is carried out by means of inert gases.

8. A method according to Claim 7, characterised in that carbon dioxide is used as the inert gas.

9. A method according to one of the preceding Claims, characterised in that the pressure comminution is carried out at a pressure of at least 30 bar, preferably at least 40 bar.

10. A method according to one of the preceding Claims, characterised in that the material which is to be comminuted is subjected to the compressed gas in a pressure chamber and is voided via an expansion valve against an impact head arranged in an expansion chamber.

11. A method according to one of the preceding Claims, characterised in that the pressure comminution is carried out in a plurality of stages.

12. An apparatus for carrying out methods according to one of the preceding Claims, characterised by at least one pressure chamber (5), ducts for material (4) to be comminuted and compressed gas (7), shut-off members (6, 8), expansion chamber (11) and pressure relief valve (10) and also a drying station (3), preferably a microwave drying station, which precedes the pressure chamber (5).

13. An apparatus according to Claim 12, characterised in that an impact head is located in the expansion chamber (11).

## Revendications

1. Procédé pour disloquer le pollen de fleurs, caractérisé en ce que l'on soumet

a) dans une première étape, le pollen de fleurs, à un séchage, et que par la suite

b) les pollens séchés subissent un traitement de fragmentation par pression, connu en soi.

2. Procédé selon la revendication 1, caractérisé en ce que dans l'étape a) le degré d'humidité des pollens est ramené à une valeur au plus égale à 8%.

3. Procédé selon l'une des revendications 1 ou 2, caractérisé en ce que la température du produit à sécher ne dépasse pas 50°C.

4. Procédé selon l'une des revendications précédentes, caractérisé en ce que l'on prévoit comme méthode de séchage, un séchage sous vide, une lyophilisation ou un séchage par micro-ondes, connus en soi.

5. Procédé selon la revendication 4, caractérisé en ce qu'il est prévu un séchage par micro-ondes.

6. Procédé selon la revendication 5, caractérisé en ce que l'on effectue le séchage pendant une durée allant jusqu'à 5 minutes.

7. Procédé selon l'une des revendications précédentes, caractérisé en ce que l'on effectue la fragmentation par pression conforme à l'étape b), au moyen de gaz inertes.

8. Procédé selon la revendication 7, caractérisé en ce que l'on utilise du dioxyde de carbone en guise de gaz inerte.

9. Procédé selon l'une des revendications précédentes, caractérisé en ce que l'on effectue la fragmentation par pression, sous une pression d'au moins 30 bar, et de préférence d'au moins 40 bars.

10. Procédé selon l'une des revendications précédentes, caractérisé en ce que l'on soumet le produit à fragmenter à un gaz sous pression, dans une chambre de pression, et que l'on vide ensuite le produit, par l'intermédiaire d'une soupape de détente, contre une tête d'impact agencée dans une chambre de détente.

11. Procédé selon l'une quelconque des revendications précédentes, caractérisé en ce que l'on effectue la fragmentation par pression, en plusieurs étapes.

12. Installation pour la mise en œuvre des procédés conformes à l'une des revendications précédentes, caractérisé par au moins une chambre de pression (5), des conduites pour le produit à fragmenter (4) et le gaz sous pression (7), des organes d'arrêt (6, 8), une chambre de détente (11) et une soupape de détente (10), ainsi qu'en supplément un poste de séchage (3), de préférence un poste de séchage par micro-ondes, qui est situé en amont de la chambre de pression (5).

13. Installation selon la revendication 12, caractérisée en ce qu'il est prévu une tête d'impact dans la chambre de détente (11).

*Fig. 1*